# EUROPÄISCHE PATENTSCHRIFT

(11) **EP 0 853 763 B1**
(45) Veröffentlichungstag und Bekanntmachung des Hinweises auf die Patenterteilung: **20.03.2002**
(21) Anmeldenummer: 96938942.8
(22) Anmeldetag: 19.09.1996
(51) Int. Cl.: G01N 33/15, G01N 13/00

(54) **VORRICHTUNG ZUM BESTIMMEN DER ZERFALLSZEIT VON VERPRESSTEN ARZNEIFORMKÖRPERN, WIE TABLETTEN, PILLEN ODER KAPSELN**
DEVICE FOR DETERMINING THE DISSOLUTION TIME OF MEDICAMENTS IN PRESSED FORM, LIKE TABLETS, PILLS OR CAPSULES
DISPOSITIF POUR LA DETERMINATION DU TEMPS DE DECOMPOSITION DE MEDICAMENTS SOUS FORME DE CORPS COMPRIMES TELS QUE PASTILLES, PILULES OU CAPSULES

(30) Priorität: 06.10.1995 DE 19537179
(43) Veröffentlichungstag der Anmeldung: 22.07.1998
(73) Patentinhaber: Löffler, Hans-Peter, 64342 Seeheim-Jugenheim (DE)
(72) Erfinder: Löffler, Hans-Peter, 64342 Seeheim-Jugenheim (DE)
(74) Vertreter: Mierswa, Klaus, Dipl.-Ing.
(86) Internationale Anmeldenummer: DE9601771
(87) Internationale Veröffentlichungsnummer: WO9714035

(56) Entgegenhaltungen:
- DE-A- 2 530 065
- DE-A- 3 325 739
- DE-A- 3 414 507
- DE-A- 3 520 034
- US-A- 3 618 395

## Beschreibung

### Technisches Gebiet:

Die Erfindung betrifft eine Vorrichtung zum Bestimmen der Zerfallszeit von verpreßten Arzneiformkörpern, wie Tabletten, Pillen oder Kapseln, bestehend aus einem Gestell mit einer Mittelsäule und mit einem Boden, der eine Mehrzahl von Löchern enthält, die von unten mit einem Netz abgedeckt sind und in denen innerhalb des Gestells Prüfröhrchen aufrecht stehend angeordnet sind, in welchen je eine Scheibe als Beschwerung des einzelnen Arzneiformkörpers beweglich einbringbar ist, gemäß dem Oberbegriff des Anspruchs 1.

### Stand der Technik:

Die Zerfallszeitmessung von verpreßten Arzneiformkörpern, wie Tabletten und Kapseln, erfolgt in einem standardisierten Versuchsaufbau, um die Reproduzierbarkeit der Meßergebnisse zu gewährleisten (insbesondere nach DAB 10, 3. Nachtrag 1994 oder Europäisches Arzneibuch oder USP[USA]). Durch die Zerfallsprüfung wird festgestellt, ob die Tabletten oder Kapseln in der vorgeschriebenen Zeit unter genau aufgeführten Bedingungen in einem flüssigen Medium zerfallen. Der Hauptteil der Apparatur besteht aus einem starren Gestell, welches sechs zylindrische Prüfröhrchen aus Glas enthält. Jedes Röhrchen ist mit einer zylindrischen Scheibe aus durchsichtigem Kunststoffmaterial genau vorgeschriebener relativer Dichte und Größe versehen. Die Prüfröhrchen werden durch eine obere und eine untere durchsichtige Platte aus Kunststoffmaterial senkrecht gehaltert, die je sechs Bohrungen haben. Alle Bohrungen haben den gleichen Abstand vom Mittelpunkt und gleichen Abstand voneinander. Auf der Unterseite der unteren Platte befindet sich ein Netz aus rostfreiem Stahldraht. In der Mitte der Platten ist eine Metallsäule so angebracht, daß die Apparatur an dieser Metallsäule in einer Aufhängevorrichtung aufgehängt und mittels eines Motors gleichmäßig 28- bis 32mal je Minute 50 bis 60 mm hoch auf- und abbewegt werden kann. Dazu wird die Apparatur in einem geeigneten Gefäß aufgehängt, welches die vorgeschriebene Flüssigkeit enthält. Nach dem Einfüllen einer Tablette oder Kapsel in jedes Röhrchen und Auflegen der Scheibe als Beschwerung erfolgt die Bestimmung der Auflösezeit der Tabletten oder Kapseln durch Beobachtung der Meßvorrichtung und Zeitnahme durch die Bedienungsperson.

Durch die DE 35 20 034 C1 ist ein Zerfallsgerät für Prüfkörper, insbesondere Tabletten, bekannt, bei dem die Prüfkörper in Behältern eines Zerfallskorbes zwischen einem Hallgenerator und einer mit einem Magneten versehenen Scheibe angeordnet sind. Die Behälter des Zerfallskorbes werden mittels einer Heizung auf eine konstante Temperatur aufgeheizt. Wenn der Prüfkörper zerfällt, bewegt sich die Scheibe gemeinsam mit dem Magneten auf den Hallgenerator zu, so daß dieser ein Signal abgibt, das nach dem Überschreiten einer Schallschwelle einem Registriergerät zugeführt und angezeigt werden kann. Die Übertragung der Energie für elektrische Schaltungen im Zerfallskorb erfolgt über Kontakte oder durch einen Hochfrequenzsender und einen Hochfrequenzempfänger. Die Übertragung der Signale zum Registriergerät erfolgt durch optoelektronische Bauteile.

Durch die DE 94 19 245 U1 ist ein automatisches Zerfallszeit-Meßgerät für die pharmazeutische Qualitäts- und Produktionskontrolle von Tabletten und Dragees innerhalb einer leitfähigen Testflüssigkeit bekannt, welches aus einem in einem Becherglas angeordneten korbartigen Gestell besteht mit einer darin angeordneten Anzahl von Glasröhren, deren Böden durch kreisförmige Siebplatten als Standflächen für die Glasröhren gebildet werden, wobei jede Siebplatte aus zwei stromdurchflossenen elektrodenbildenden Drahtgeflechtshälften besteht, die unter Ausbildung eines Schlitzes in Abstand voneinander angeordnet sind. In jeder Glasröhre wird ein Prüfling angeordnet, der mittels eines Schwimmers abgedeckt ist, der auf dem Prüfling aufliegt. Der Schwimmer weist auf der Unterseite ein eingebettetes Kontaktgerüst aus einem metallischen Werkstoff auf. Bei Bewegung des Becherglases und Zerfall der Tabletten ändert sich die Leitfähigkeit der Testflüssigkeit, die zwischen den Drahtgeflechtshälften und dem Kontaktgerüst des Schwimmers gemessen wrden kann.

Durch die US 3,618,395 ist Tabletten-Zerfallszeitmeßgerät mit bewegten Röhrchen in einer Badflüssigkeit bekannt mit einer Vielzahl von sich gegenüberliegenden beabstandeten Elektroden auf dem Boden der die Tabletten enthaltenden Röhrchen. Die Gegenwart einer Tablette stört ein an die Elektroden angelegtes elektromagnetisches Feld, wobei die Störung einen Zeitgeber beeinflusst, dessen Signale ausgewertet werden können.

Bisherige Ansätze für eine vollautomatische Messung gingen immer mit nicht zulässigen Änderungen der Versuchsapparatur einher. Derartige Änderungen sind jedoch gemäß DAB 10 nur in sehr geringem Umfang zulässig.

### Technische Aufgabe:

Der Erfindung liegt die Aufgabe zugrunde, eine Vorrichtung der eingangs genannten Gattung zu schaffen, die ohne Änderung der vorgeschriebenen Parameter für die Apparatur gemäß DAB 10 berührungslos die Bewegung der Scheiben innerhalb der Vorrichtung erfassen und kontinuierlich die beim Auflösungsvorgang abnehmende Dicke oder die Restdicke von verpreßten Arzneiformkörpern, wie Tabletten, Pillen oder Kapseln, ermitteln soll. Des weiteren sollen die Scheiben und Gestelle untereinander austauschbar sein, ohne eine Neukalibrierung der Vorrichtung durchführen zu müssen; die Scheiben und Gestelle sollen wie bisher einfach zu reinigen sein, ohne daß dadurch das Meßsystem beeinflußt wird.

### Offenbarung der Erfindung und deren Vorteile:

Die Lösung der Aufgabe besteht erfindungsgemäß darin, daß auf dem und/oder im Boden des Gestells um jedes Loch eine elektrische Spule angeordnet ist, die Teil eines elektrischen Schwingkreises ist, wobei auf und/oder in der Scheibe eine Leiterschleife zur wegabhängigen Dämpfung des elektrischen Schwingkreises angeordnet ist, die gemeinsam an eine elektrische Versorgungs- und Auswerteeinrichtung zur Schwingungserzeugung und Auswertung der Meßergebnisse angeschlossen sind. Die Spule kann als Einlagen- oder Mehrlagenspule ausgebildet sein.

Normalerweise wird die Restdicke als Sollwert vorgegeben. Wenn der Istwert des Arzneiformkörpers ≤ der Restdicke ist, so gilt derselbe als aufgelöst.

In vorteilhafter Weise ist die Vorrichtung so gestaltet, daß die Geometrie und Dichte der Scheiben sowie die Geometrie der Prüfröhrchen und der Aufbau des Siebbodens des Gestells unverändert ist. Ebenso sind die Scheiben und die Gestelle untereinander austauschbar, ohne daß eine Neukalibrierung der Vorrichtung durchgeführt werden muß.

Vorteilhaft kann die Spule als Einlagen- oder Mehrlagenspule auf einem separaten Spulenträger ausgebildet sein, der entweder auf dem Boden des Gestells sitzt oder integral mit dem Boden des Gestells ausgeführt ist oder identisch mit dem Boden des Gestells sein kann. Jede Spule kann eine Flachspule sein, wobei die eine Hälfte der Windungen der Flachspule auf der Oberseite und die andere Hälfte auf der Unterseite des Bodens bzw. des Spulenträgers integriert sind und die Zuleitungen zu den Spulen bezüglich der Ober- und Unterseite des Bodens bzw. des Spulenträgers deckungsgleich ausgeführt sind. Ebenso können die Spulen in Multilayer-Technik auf einer Multilayer-Platine als Boden bzw. als Spulenträger angeordnet sein wie auch die Spulen innerhalb eines vom Boden des Gestells separierten Spulenbodens bzw. Spulenträgers angeordnet sein können.

Eine gegenseitige Beeinflussung der Meßstellen bzw. der Schwingkreise wird dadurch vermieden, daß die elektrischen Schwingkreise mittels eines Multiplexers ansteuerbar sind, der jeweils nur einen Schwingkreis erregt.

Die Energieversorgung und Datenübertagung erfolgt bevorzugt kontaktlos durch ein magnetisches Feld zwischen Gestell und einer Antriebseinheit, innerhalb der das Gestell mittels der Mittelsäule gehaltert ist.

Des weiteren kann die elektrische Koppelstrecke aus jeweils einer Hälfte eines Schalenkerns mit eingelegter Spule auf jeder Seite bestehen, wobei auf der Antriebsseite die Schalenkernhälfte mit Spule in die Halterung für das Gestell bzw. für die Mittelsäule und auf der Seite des Gestells die Schalenkernhälfte mit Spule in die Mittelsäule integriert ist.

Die Sensoren, bestehend aus Schwingkreise, Auswerteeinrichtung sowie induktiver Energie- und Datenkopplung können vorzugsweise an einen gemeinsamen Bus geschaltet sein und eine Busadresse erhalten, wobei in die zu übertragende Nachricht eine Teilnehmeradresse eingefügt wird zum Parallelbetrieb der Sensoren am Bus. Zur Adressvergabe sind die Sensoren einzeln an den Bus aufschaltbar. Die Sende- und Empfangsspulen der Sensoren zum Ankoppeln an die Antriebseinheit können zur Trennung des Energie- und Datenflusses mit unterschiedlichen Frequenzen betrieben werden.

Eine Änderung der Dichte der Scheiben durch das Einlegen der Leiterschleife wird, falls es notwendig sein sollte, durch einen Hohlraum im Innern der Scheibe ausgeglichen.

Vorteilhaft werden die Schwingkreisspulen durch Leiterbahnen innerhalb des Bodens gebildet, die das jeweilige Prüfröhrchen umschließen. Durch die geringe Länge der Spulen ist die räumliche Ausdehnung des Feldes so begrenzt, daß eine Beeinflussung des Schwingkreises durch den Siebboden des Gestells gering ist. Eine gegenseitige Beeinflussung der Meßstellen wird durch den Einsatz des Multiplexers vermieden.

### Kurzbeschreibung der Zeichnung, in der zeigen:

- Figur 1 a,b,c: eine Vorrichtung des Standes der Technik gemäß DAB 10
- Figur 2: ein Boden mit darauf ausgebildeten Spulen um die Löcher zum Einstecken der Prüfröhrchen zur Ausbildung von Schwingkreisen
- Figur 3: eine Scheibe, wie sie in jedes Prüfröhrchen eingesetzt wird
- Figur 4: ein Blockschaltbild des elektrischen Teils der Vorrichtung Figur 5 ein Blockschaltbild eines Gesamtsystems mit an einen Bus angeschlossene vier Vorrichtungen oder Sensoren und
- Figur 6: ein Blockschaltbild des Powermodems.

### Wege zur Ausführung der Erfindung:

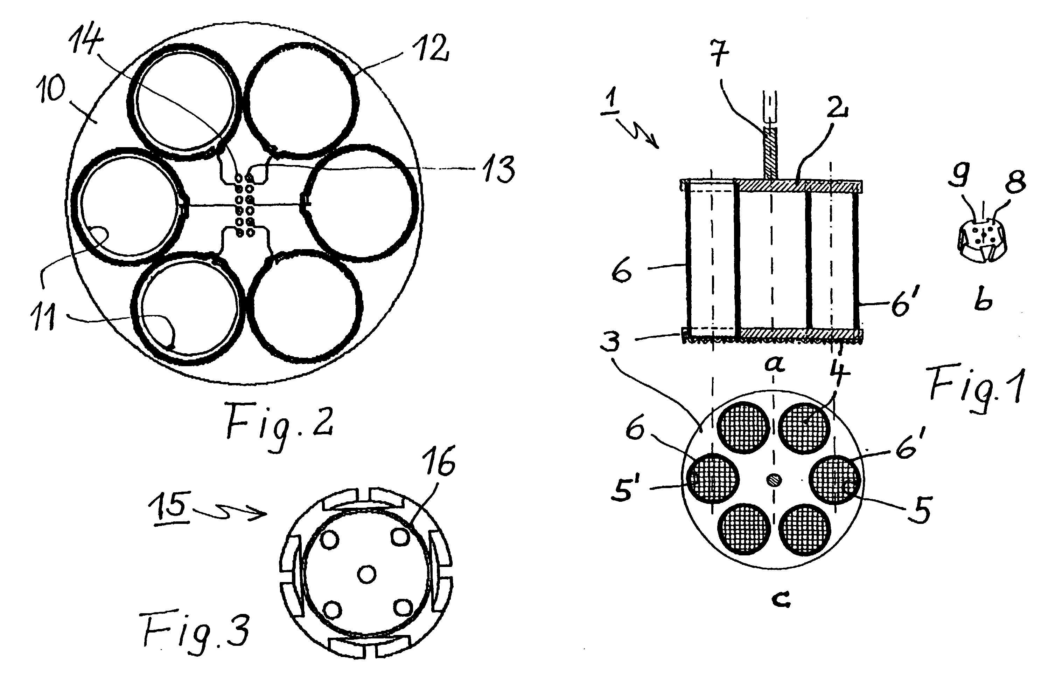

Die Figur 1 a, b und c zeigt eine Vorrichtung gemäß DAB 10,3. Nachtrag 1994. Die Vorrichtung besteht aus einem Gestell 1 mit einer Mittelsäule 7 sowie mit einer kreisrunden Abdeckplatte 2 und einem Boden 3, die eine Mehrzahl von Löchern 5, 5', in der Regel sechs, aufweisen, in denen Prüfröhrchen 6, 6' aus Glas angeordnet sind. Der Boden 3 ist auf der Unterseite mit einem Netz 4 aus rostfreiem Stahldraht abgedeckt. Die Platten 2, 3 sind voneinander durch nichtgezeigte senkrechte Metallstäbe an der Außenseite des Gestells 1 starr gehalten. Zur Zerfallsprüfung wird jeweils eine Tablette oder Kapsel in jedes Prüfröhrchen 6,6' gelegt und eine Scheibe 8, die genau definierte Aussparungen 9 besitzt, auf die Tablette oder Kapsel als Beschwerung aufgelegt und die Zerfallszeit gemessen.

In den Figuren 2 und 3 ist die mechanische Abwandlung der Teile gezeigt, die gegenüber der Apparatur gemäß DAB 10 verändert sind.

Auf einem Boden 10, der dem Boden 3 der Figur 1 entspricht, sind Löcher 11 angeordnet, die den gleichen Abstand vom Mittelpunkt des Bodens 10 sowie gleichen Abstand voneinander haben. Um die Löcher 11 ist jeweils eine Spule 12 angeordnet, deren Windungsendungen zu Kontakten 13, 14 führen; der Boden stellt somit den Spulenträger 10 dar; die Spulen 12 sind in den Boden integriert. Der Spulenträger 10 kann jedoch auch als Zwischenboden ausgeführt sein.

Eine jede Spule ist als Flachspule 12 ausgeführt, wobei die eine Hälfte der Windungen der Flachspule auf der Oberseite und die andere Hälfte auf der Unterseite des Bodens 10 kontaktiert sind und die Zuleitungen 13, 14 zu den Spulen bezüglich der Ober- und der Unterseite des Bodens 10 deckungsgleich ausgeführt sind. Die Windungsendungen 13, 14 führen oberhalb und unterhalb des Bodens zu Kontaktstiften eines Steckers gemäß Figur 2; die Leitungen werden in der Mittelsäule 7 nach oben geführt.

Eine Scheibe 15, die in ihren Abmessungen und ihrer Dichte der Scheibe 8 gemäß DAB 10 entspricht, weist auf einer Oberfläche oder integriert eine Leiterschleife 16 auf.

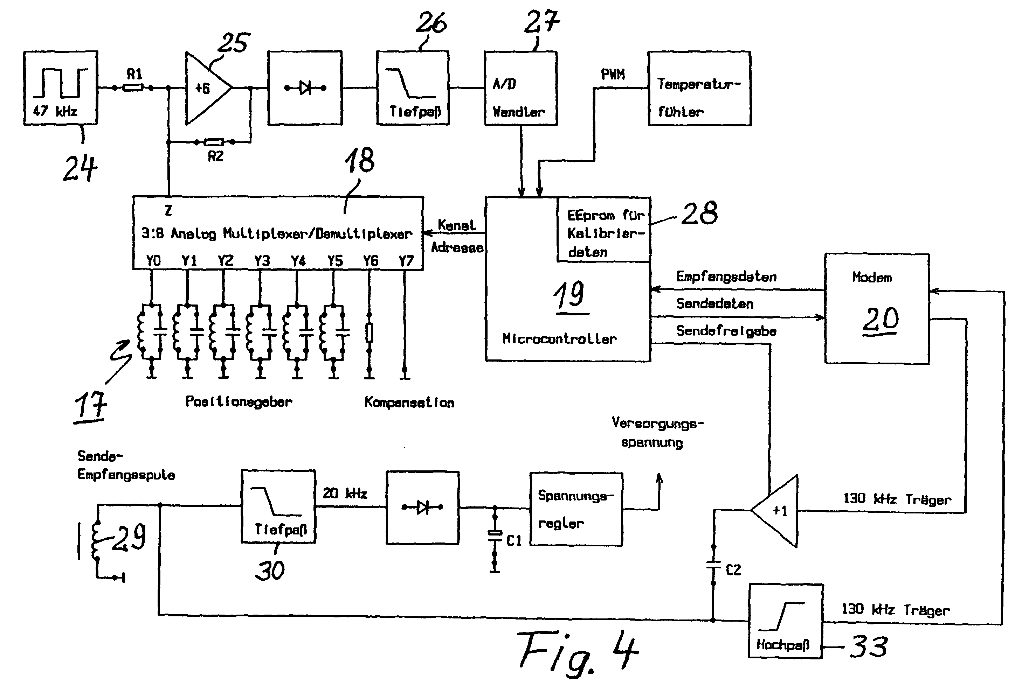

Gemäß Figur 4 sind die Spulen 12 jeweils Teil von LC-Schwingkreisen 17, die über einen Analog-Multiplexer 18 von einem Rechteckgenerator 24 nacheinander angeregt werden. Durch die in der Scheibe 15 befindliche Leiterschleife 16 wird der einzelne Schwingkreis wegabhängig bedämpft. Da die Resonanzfrequenz des Schwingkreises 17 sehr viel höher gewählt ist als die Erregerfrequenz des Rechteckgenerators 24, erfolgt nach jeder Flanke des Erregersignals eine abklingende Schwingung des Schwingkreises 17. Je stärker die Dämpfung des Schwingkreises 17 ist, desto schneller klingt die Schwingung ab. Damit stellt die Fläche der Hüllkurve ein Maß für die Dämpfung des Schwingkreises 17 dar.
Die Realisierung der Meßwerterfassung erfolgt durch Zuführen der Rechteckspannung des Rechteckgenerators 24 über den Analog-Multiplexer 18 an die LC-Schwingkreise 17 der Vorrichtung. Die sich innerhalb der Schwingkreise 17 ergebende Spannung wird wiederum über den Multiplexer 18 einem Verstärker 25 zugeführt. Durch eine Mitkopplung des Verstärkerausgangs auf den Eingang können Eigenverluste der Schwingkreise 17 zum Teil ausgeglichen werden.

Die Ermittlung der Hüllkurvenfläche der Schwingkreisspannung erfolgt durch Gleichrichtung und anschließende Tiefpaßfilterung 26 der verstärkten Schwingkreisspannung. Die Ausgangsspannung des Tiefpaßfilters 26 wird einem A/D-Wandler 27 zugeführt, dessen Ausgangssignal einem Mikrocontroller 19 aufgegeben wird, der aus dem Spannungswert den Abstand der entsprechenden Scheibe 15 zum Boden des Gestells 1 errechnet. Korrekturfaktoren können von der Software des Mikrocontrollers 19 in einem Kalibriervorgang selbsttätig ermittelt und in einen nichtflüchtigen Speicher 28, der vorzugsweise ein EEprom ist, abgelegt werden.

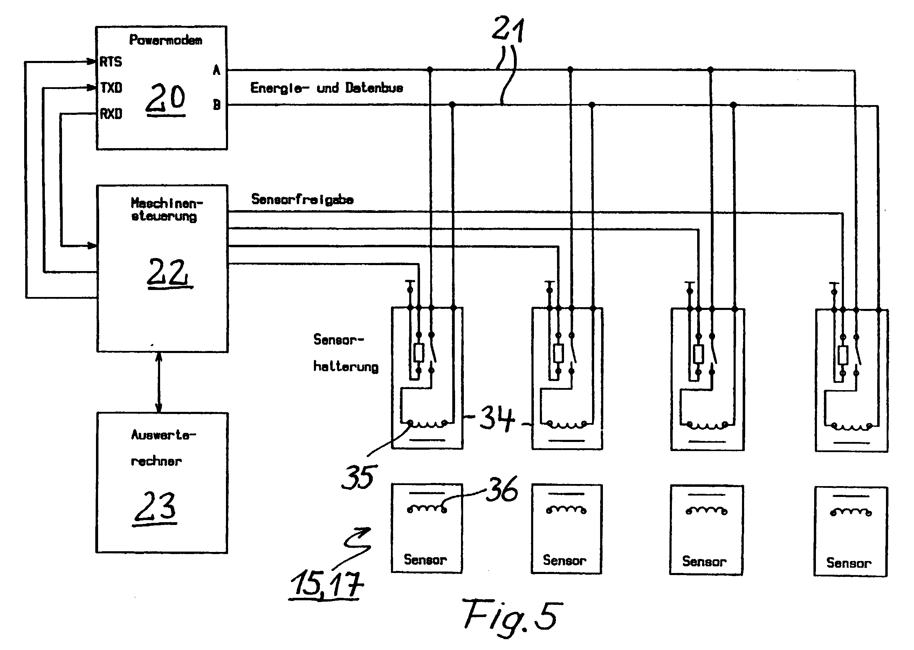

Während der Zerfallszeitbestimmung der Tabletten oder Kapseln werden die Abstandswerte kontinuierlich erfaßt und an einen Auswerterechner 23, Figur 5, über eine induktive Koppelstrecke übertragen.

Gemäß Figur 5 erfolgt die Energieversorgung und Datenübertragung zwischen Gestell 1 und der Auswerteeinrichtung 23 vorzugsweise kontaktlos durch ein magnetisches Feld. Dazu besteht die elektrische Koppelstrecke aus jeweils einer Hälfte eines Schalenkerns mit eingelegter Spule 35 auf der Seite der Sensorhalterung 34, das ist die Antriebsseite für das Gestell, sowie einer Schalenkernhälfte mit Spule 36 auf der Sensorseite. In der Sensorhalterung 34 sind Schalenkern und Spule 35 in die Halterung für das Gestell integriert, auf der Seite des Gestells 1 bzw. des Sensors sind Schalenkernhälfte und Spule 36 in die Mittelsäule 7 des jeweiligen Gestells 7 integriert.

Die Energie- und Datenübertragung an die Spulen 12 kann gleichzeitig über die Koppelstrecke 35-36 mit unterschiedlichen Frequenzen erfolgen, wodurch eine Trennung der Energie- und Datenübertragung mittels Filter möglich ist. Die Energieübertragung erfolgt durch Anlegen einer 20 kHz-Wechselspannung eines Sinusgenerators 31 an die Primärseite einer Sende-Empfangsspule 29 der Koppelstrecke bzw. des Übertragers. Die senkundärseitig induzierte Spannung wird nach einem Tiefpaßfilter 30 zur Abtrennung des Datensignals gleichgerichtet und stabilisiert.

Aus Figur 5 ist des weiteren der Anschluß der Sensorhalterungen 34 an den Energie- und Datenbus 21 ersichtlich. Die Sensoren, bestehend jeweils aus Schwingkreise 17, Auswerteeinrichtung 20, 23 sowie induktiver Energie- und Datenkopplung mittels der Koppelstrecke 35-36, sind an einen gemeinsamen Bus 21 geschaltet und können eine Busadresse erhalten, wozu in die zu übertragende Nachricht eine Teilnehmeradresse eingefügt wird.

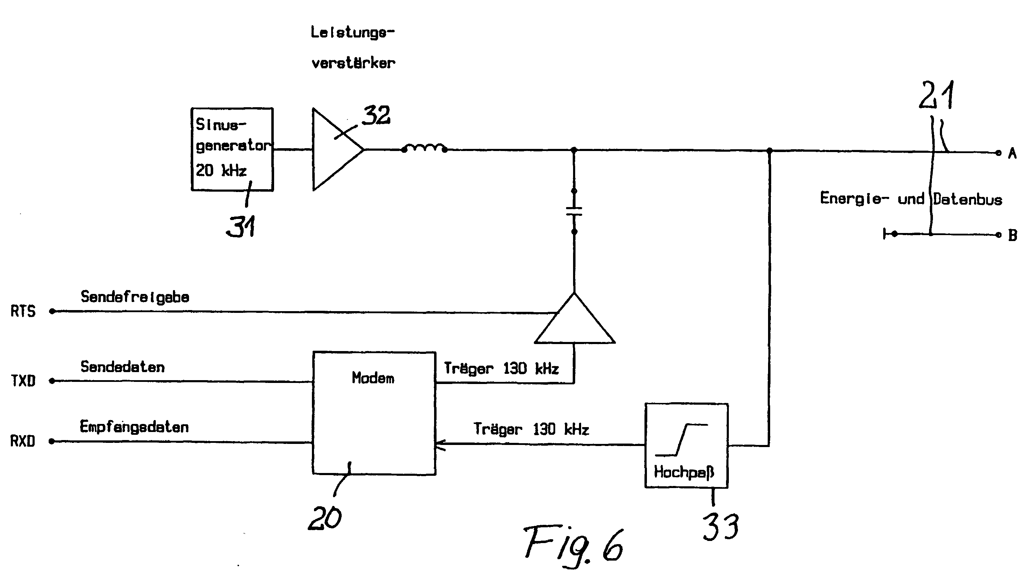

Die Datenübertragung kann gemäß der Figuren 5 und 6 halbduplex durch ein Modem 20 auf jeder Seite der Koppelstrecke erfolgen. Der jeweilige Datensender moduliert eine Trägerfrequenz, vorzugsweise in AM oder in FSK, von 130 kHz und schaltet das Signal über einen Leistungsverstärker 32 auf die Wicklung des Übertragers auf. Die Empfängerseite führt die Ausgangsspannung des Übertragers über einen Hochpaßfilter 33 dem Eingang des Modems 20 zu. Das Modem 20 demoduliert das Signal und gewinnt den Bitstrom des Senders zurück.

Durch das Einfügen von Teilnehmeradressen in die Nachrichten ist der parallele Betrieb mehrerer Vorrichtung bzw. Gestelle an einem Powermodem 20 möglich. Die Vorrichtungen werden zuerst einzeln an den Bus 21 angeschaltet und erhalten eine Busadresse. Nachdem alle Vorrichtungen eine Adresse erhalten haben, erfolgt der Parallelbetrieb am Bus.

### Gewerbliche Anwendbarkeit:

Die Vorrichtung ist insbesondere zum Bestimmen der Zerfallszeit von verpreßten Arzneiformkörpern gemäß DAB, Europäisches Arzneibuch oder USP gewerblich anwendbar. Die Nützlichkeit und die Vorteile der Erfindung liegen insbesondere darin, daß mit derselben eine berührungslose und kontinuierliche Erfassung der Bewegung der Scheiben und somit des Zerfalls von verpreßten Arzneiformkörpern, wie Tabletten, Pillen oder Kapseln, innerhalb der Prüfröhrchen möglich ist, um daraus die momentane Dicke des Arzneiformkörpers beim Auflösevorgang und die Zerfallszeit elektrisch zu bestimmen.

### Liste der Bezugszeichen:

- 1: Gestell
- 2: Abdeckplatte
- 3: Boden
- 4: Netz
- 5,5': Löcher
- 6.6': Prüfröhrchen
- 7: Mittelachse
- 8: Scheibe
- 9: Aussparungen
- 10: Spulenträger oder Boden
- 11: Löcher
- 12: Spulen
- 13,14: Kontakte
- 15: Scheibe
- 16: Leiterschleife
- 17: Schwingkreise
- 18: Multiplexer
- 19: Mikrocontroller
- 20: Modem bzw. Powermodem
- 21: Bus
- 22: Maschinensteuerung
- 23: Auswerterechner
- 24: Rechteckgenerator
- 25, 32: Verstärker
- 26: Tiefpaßfilter
- 27: A/D-Wandler
- 28: nichtflüchtiger Speicher
- 29: Sende-Empfansspule
- 30: Tiefpaßfilter
- 31: Sinusgenerator
- 33: Hochpaßfilter
- 34: Sensorhalterungen
- 35, 36: Koppelspulen

## Patentansprüche

1. Vorrichtung zum Bestimmen der Zerfallszeit von verpreßten Arzneiformkörpern, wie Tabletten und Kapseln, bestehend aus einem Gestell (1) mit einer Mittelsäule (7) und einem Boden (3,10), der eine Mehrzahl von Löchern (5,5',11), enthält, die von unten mit einem Netz (4) abgedeckt sind und in denen innerhalb des Gestells (1) Prüfröhrchen (6,6') aufrecht stehend angeordnet sind, in welchen je eine Scheibe (8,15) als Beschwerung des einzelnen Arzneiformkörpers beweglich einbringbar ist, **dadurch gekennzeichnet,**
**daß** auf dem und/oder im Boden (10) um jedes Loch (11) eine elektrische Spule (12) angeordnet ist, die Teil eines elektrischen Schwingkreises (17) ist, wobei auf und/oder in der Scheibe (15) eine Leiterschleife (16) zur wegabhängigen Dämpfung des elektrischen Schwingkreises (17) angeordnet ist, die gemeinsam an eine elektrische Versorgungs- und Auswerteeinrichtung (20,23) zur Schwingungserzeugung und Auswertung der Meßergebnisse angeschlossen sind.

2. Vorrichtung nach Anspruch 1, **dadurch gekennzeichnet,**
**daß** die Spule (12) als Einlagen- oder Mehrlagenspule ausgebildet ist.

3. Vorrichtung nach Anspruch 1 oder 2, **dadurch gekennzeichnet,**
**daß** jede Spule eine Flachspule (12) ist, wobei die eine Hälfte der Windungen der Flachspule (12) auf der Oberseite und die andere Hälfte auf der Unterseite eines Spulenträgers (10) bzw. des Bodens (3) des Gestells (1) integriert sind und die Zuleitungen zu den Spulen bezüglich der Ober- und Unterseite des Spulenträgers (3,10) bzw. des Bodens (3) deckungsgleich ausgeführt sind.

4. Vorrichtung nach Anspruch 1 oder 2, **dadurch gekennzeichnet,**
**daß** die Spulen (12) in Multilayer-Technik auf einer Multilayer-Platine als Spulenträger bzw. Boden angeordnet sind.

5. Vorrichtung nach Anspruch 1 oder 2, **dadurch gekennzeichnet,**
**daß** die Spulen innerhalb eines vom Boden des Gestells separierten Spulenbodens (10) oder Spulenträgers angeordnet sind.

6. Vorrichtung nach nach Anspruch 1 oder 2, **dadurch gekennzeichnet,**
**daß** die elektrischen Schwingkreise (17) mittels eines Multiplexers (18) ansteuerbar sind.

7. Vorrichtung nach nach Anspruch 1 oder 2, **dadurch gekennzeichnet,**
**daß** die Energieversorgung und Datenübertagung kontaktlos durch ein magnetisches Feld zwischen Gestell (1) und einer Antriebseinheit erfolgt, innerhalb der das Gestell (1) mittels der Mittelsäule (7) gehaltert ist.

8. Vorichtung nach Anspruch 7, **dadurch gekennzeichnet,**
**daß** die elektrische Koppelstrecke aus jeweils einer Hälfte eines Schalenkerns mit eingelegter Spule (35,36) auf jeder Seite besteht, wobei auf der Antriebsseite die Schalenkernhälfte mit Spule (35) in die Halterung für das Gestell (1) bzw. für die Mittelsäule (7) und auf der Seite des Gestells die Schalenkernhälfte mit Spule (36) in die Mittelsäule (7) integriert ist.

9. Vorrichtung nach Anspruch 1 oder 2, **dadurch gekennzeichnet,**
**daß** eine Mehrzahl von Gestellen (1) bzw. Sensoren, bestehend aus Schwingkreise (17), Auswerteeinrichtung (20,23) sowie induktiver Energie- und Datenkopplung, an einen gemeinsamen Bus (21) geschaltet sind und eine Busadresse erhalten und in die zu übertragende Nachricht eine Teilnehmeradresse eingefügt wird zum Parallelbetrieb der Sensoren am Bus.

10. Vorrichtung nach den Ansprüchen 7 und 9, **dadurch gekennzeichnet,**
**daß** die Sende- und Empfangsspulen (35,36) der Sensoren zum Ankoppeln an die Antriebsseite der Gestelle (1) zur Trennung des Energie- und Datenflusses mit unterschiedlichen Frequenzen betrieben werden.

11. Verwendung der Vorrichtung nach einem der Ansprüche 1 bis 10 zum Bestimmen der Zerfallszeit von verpreßten Arzneiformkörpern gemäß DAB, Europäisches Arzneibuch oder USP.

## Claims

1. A device for determining the disintegration time of compressed drug products such as tablets and capsules, consisting of a rack (1) with a middle column (7) and a base (3, 10) having a plurality of holes (5, 5', 11) that are covered by a net (4) on the bottom and in which test tubes (6, 6') are arranged upright inside the rack (1), whereby a disk (8, 15) that serves to weigh down the individual drug product can be movably placed into said test tubes, **characterized in that**
on and/or in the base (10), around each hole (11), there is an electric coil (12) that is part of an electric oscillating circuit (17), whereby on and/or in the disk (15), there is a conductor loop (16) for the path-dependent attenuation of the electric oscillating circuit (17), said electric coils being jointly connected to an electric supply and evaluation unit (20, 23) for generating oscillations and evaluating the measured results.

2. The device according to Claim 1, **characterized in that**
the coil (12) is configured as a single-layer or multi-layer coil.

3. The device according to Claim 1 or 2, **characterized in that**
each coil is a flat coil (12), whereby one half of the windings of the flat coil (12) are integrated on the top and the other half are integrated on the bottom of a coil carrier (10) or of the base (3) of the rack (1) and the feed lines to the coils are configured to as to be congruent relative to the top and bottom of the coil carrier (3, 10) or of the base (3).

4. The device according to Claim 1 or 2, **characterized in that**
a multi-layer technique is used to arrange the coils (12) on a multi-layer printed circuit board as the coil carrier or base.

5. The device according to Claim 1 or 2, **characterized in that**
the coils are arranged inside a coil base (10) or a coil carrier that is separated from the base of the rack.

6. The device according to Claim 1 or 2, **characterized in that**
the electric oscillating circuits (17) can be actuated by means of a multiplexer (18).

7. The device according to Claim 1 or 2, **characterized in that**
the energy supply and data transmission take place contact-free by means of a magnetic field between the rack (1) and a drive unit, inside which the rack (1) is held by means of the middle column (7).

8. The device according to Claim 7, **characterized in that**
the electric coupling path consists of one half of a pot core with an inserted coil (35, 36) on each side, whereby on the drive side, the pot core half with the coil (35) is integrated into the holder for the rack (1) or for the middle column (7), and on the side of the rack, the pot core half with the coil (36) is integrated into the middle column (7).

9. The device according to Claim 1 or 2, **characterized in that**
a plurality of racks (1) or sensors, consisting of oscillating circuits (17), evaluation means (20, 23) as well as inductive energy and data coupling, are connected to a joint bus (21) and receive a bus address, and a participant address is inserted into the message to be transmitted for the parallel operation of the sensors on the bus.

10. The device according to Claims 7 and 9, **characterized in that**
the transmitting and receiving coils (35, 36) of the sensors for coupling to the drive side of the racks (1) used for separating the energy and data flow are operated at different frequencies.

11. The use of the device according to one of Claims 1 through 10 for determining the disintegration time of compressed drug products in accordance with the German Pharmacopoeia (DAB), the European Pharmacopoeia (Europäische Arzneibuch) or the USP.

## Revendications

1. Dispositif pour déterminer le temps de désagrégation de corps moulés de médicaments compactés tels que des comprimés ou des capsules, comprenant un support (1) avec une colonne centrale (7) et un fond (3, 10) qui comporte une pluralité de trous (5, 5', 11) recouverts par le bas par un filet (4) et dans lesquels sont placées toutes droites à l'intérieur du support (1) des éprouvettes (6, 6') dans chacune desquelles un disque (8, 15) peut être introduit de façon mobile comme poids de charge de chacun des corps de médicaments moulés, **caractérisé en ce que**
sur et/ou dans le fond (10), est disposée autour de chaque trou (11) une bobine électrique (12) qui fait partie d'un circuit oscillant électrique (17), une boucle conductrice (16) pour l'amortisation du circuit oscillant électrique (17) asservie à la course étant disposée sur et/ou dans le disque (15) et connectée, avec la bobine, à une unité d'alimentation électrique et d'analyse (20, 23) destinée à la génération d'oscillations et à l'analyse des résultats de mesure.

2. Dispositif selon la revendication 1, **caractérisé en ce que**
la bobine (12) est conformée par une couche ou par plusieurs couches.

3. Dispositif selon l'une des revendications 1 ou 2 **caractérisé en ce que**
chaque bobine est une bobine plate (12), la moitié des spires de la bobine plate (12) étant intégrée sur le côté du dessus et l'autre moitié sur le côté du dessous d'un support de bobine (10) ou d'un fond (3) du support (1) et que les conducteurs vers les bobines coïncident par rapport aux côtés du dessus et du dessous du support de bobine (3, 10) ou du fond (3).

4. Dispositif selon l'une des revendications 1 ou 2, **caractérisé en ce que**
les bobines (12) en technique multicouches sont disposées sur une platine multicouche faisant office de support de bobine ou de fond.

5. Dispositif selon l'une des revendications 1 ou 2, **caractérisé en ce que**
les bobines sont agencées à l'intérieur d'un fond de bobine (10) ou d'un support de bobine séparé du fond du support.

6. Dispositif selon l'une des revendications 1 ou 2, **caractérisé en ce que**
les circuits oscillants électriques (17) peuvent être commandés au moyen d'un multiplexeur (18).

7. Dispositif selon l'une des revendications 1 ou 2, **caractérisé en ce que**
l'alimentation en énergie et la transmission de données se produisent sans contact par un champ magnétique entre le support (1) et une unité d'entraînement à l'intérieur de laquelle le support (1) est maintenu au moyen de la colonne centrale (7).

8. Dispositif selon la revendication 7, **caractérisé en ce que**
le segment de couplage électrique est composé respectivement d'une moitié de noyau de coquille avec bobine insérée (35,36) de chaque côté, la moitié de noyau de coquille avec la bobine (35) côté entraînement étant intégrée dans la fixation pour le support (1) ou pour la colonne centrale (7), et du côté du support, la moitié du noyau de coquille avec la bobine (36) étant intégrée dans la colonne centrale (7).

9. Dispositif selon l'une des revendications 1 ou 2, **caractérisé en ce que**
une pluralité de supports (1) ou de capteurs se composant de circuits oscillants (17), d'une unité d'analyse (20, 23), ainsi que d'un couplage inductif d'énergie et de données sont connectés à un bus commun (21) et obtiennent une adresse bus, et qu'une adresse destinataire est jointe au message à transmettre pour l'opération en parallèle des capteurs sur le bus.

10. Dispositif selon les revendications 7 et 9, **caractérisé en ce que**
les bobines émettrices et réceptrices (35, 36) des capteurs pour le couplage au côté entraînement des supports (1) travaillent à des fréquences différentes pour la séparation du flux d'énergie et du flux des données.

11. Utilisation du dispositif selon l'une quelconque des revendications 1 à 10 pour la détermination du temps de désagrégation de corps moulés de médicaments compactés selon DAB (pharmacopée allemande), la pharmacopée européenne ou l'USP.
